# EUROPEAN PATENT APPLICATION

(11) **EP 2 194 117 A1**
(43) Date of publication of application: **09.06.2010**
(21) Application number: 08170493.4
(22) Date of filing: 02.12.2008
(51) Int. Cl.: C12M 1/00

(54) **Photoreactor**

(71) Applicant: Reddy-Solutions, 1703 RB Heerhugowaard (NL)
(72) Inventor: Lommerts, Bert Jan, 1703 RB Heerhugowaard (NL); Bastiaansen, Cornelis Wilhelmus Maria, 6065 EE Montfort (NL); Plasschaert, Helena, 3571 ZZ Utrecht (NL); Shufen, Tsoi, 6006 LA Weert (NL)
(74) Representative: Oberlein, Gerriet H. R.

(57) **Abstract**

A photoreactor is provided comprising at least one reaction vessel, wherein the at least one reaction vessel exhibits a trapezoidal cross-section, a left lateral face (1), a right lateral face (2), a top lateral face (3), and a bottom lateral face (4), and wherein at least one of the said faces (1), (2), (3) and (4) is light-transmissive.
The photoreactor exhibits an enhanced uniformity of the light intensity distribution from the light transmissive wall of the photoreactor as far to those volume elements of the photoreactor which are farthest from the light-transmissive wall.

## Description

The present invention pertains to a photoreactor.

Photoreactors are devices, wherein phototropic microorganisms like algae are cultured, i.e. either grown and proliferated or used to produce certain substances with the aid of phototropic cells. The chemical reactions which are intended to take place in a photoreactor need - inter alia - light. To achieve a high yield of the intended reactions it is desirable to establish the same light intensity at an absolute value throughout the volume of the photoreactor, which is as close as possible to the intensity of the incident light that is used to illuminate the reactor.

Normally, the light used to illuminate a photoreactor comes into the reactor by passing a light-transmissive wall of said reactor. However, light which has passed a light-transmissive wall of a photoreactor loses its initial intensity on its path inside the reactor resulting in a decreasing gradient of the rate of the running reactions as a function of the distance to the light-transmissive wall.

Such a gradient is undesired because after a certain reaction time said gradient causes a sufficiently high concentration of the desired reaction products directly under the light-transmissive wall, while in the reactor volume that is most remote from the light-transmissive wall, the concentration of the desired products is low. Consequently, only a small percentage of the reactor volume exhibits the highest possible reaction rate, whereas in the reactor volume remote from the light-transmissive wall, only a fraction of the initial light intensity arrives and hence, merely a fraction of the desired reaction takes place.

Therefore, the problem underlying the present invention is to provide a photoreactor which exhibits an enhanced uniformity of the light intensity distribution from the light transmissive wall of the photoreactor to those volume elements of the photoreactor, which are farthest away from the light-transmissive wall.

This problem is solved by a photoreactor comprising at least one reaction vessel, wherein the at least one reaction vessel exhibits a trapezoidal cross-section, a left lateral face (1), a right lateral face (2), a top lateral face (3), and a bottom lateral face (4), and wherein at least one of the said faces (1), (2), (3) and (4) is light-transmissive.

Surprisingly, the inventive photoreactor exhibits an enhanced uniformity of the light intensity distribution from the light-transmissive wall of the photoreactor as far to those volume elements of the photoreactor that are farthest from the light-transmissive wall.

At least one of the faces (1), (2), (3), and (4) of the inventive photoreactor is light-transmissive. Within the scope of the present invention, this means that light passes through at least one face directly or diffusively. So, at least one of said faces (1), (2), (3), and (4) of the inventive photoreactor may consist of a material, which allows the light that is used to illuminate the inventive photoreactor - e.g. solar light or artificially generated light - pass directly or diffusively. Consequently, such materials are preferred to form at least one of said faces (1), (2), (3) and (4), which exhibit a high degree of light-transmissivity like glass and polymethyl methacrylate.

In a preferred embodiment of the inventive photoreactor the top lateral face (3) is light-transmissive.

In another preferred embodiment of the inventive photoreactor each of the said faces (1), (2), (3), and (4) is light-transmissive.

In one embodiment the inventive photoreactor comprises at least one reaction vessel having the properties mentioned above, wherein it's lateral faces (1) to (4) consist of a face-material, which is light-transmissive for at least one of said faces.

In a preferred embodiment of the inventive photoreactor, the at least one reaction vessel is completely contained within a support structure, which has a cross-section in the shape of a parallelogram, wherein the support structure exhibits a left lateral face (5), a right lateral face (6), a top lateral face (7), and a bottom lateral face (8) and wherein said support structure consists of a light-transmissive material. In this embodiment the lateral faces (1) to (4) of the at least one reaction vessel are realized by at least one cavity, wherein each cavity exhibits a trapezoidal cross-section.

In a further preferred embodiment of the inventive photoreactor, the trapezoidal cross-section of the at least one reaction vessel exhibits edges A, B, C and D, wherein the edges A and B are connected by a side AB, the edges C and D are connected by a side CD, the edges A and C are connected by a side AC, the edges B and D are connected by a side BD, the side AB is parallel to the side CD and the side AC is not parallel to the side BD. So, in this embodiment of the inventive photoreactor, the angle α formed by the sides AB and AC differs from the angle β formed by the sides AB and BD.

In an especially preferred embodiment of the inventive photoreactor, the side AC has a length L_{AC}, the side BD has a length L_{BD}, wherein L_{AC} = L_{BD}. So, in this embodiment of the inventive photoreactor the angle α equals the angle β.

In an even more preferred embodiment of the inventive photoreactor, the side AC and the side BD are shaped in the form of a number of steps, wherein the number of steps of side AC is n_{AC}, and the number of steps on side BD is n_{BD}.

In the embodiment of the inventive photoreactor mentioned directly above, it is preferred that n_{AC} = n_{BD} = n, wherein n preferably is from 2 to 50 · 10⁶, even more preferably from 5 to1000 and most preferably from 3 to 100.

In a further preferred embodiment of the inventive photoreactor, at least one of said faces (1), (2), (3), (4), (5), (6), (7) and (8) is provided with a scatterer, which preferably is predominantly a forward scatterer.

In a further preferred embodiment of the inventive photoreactor, the top lateral face (7) is provided with a scatterer, which preferably is predominantly a forward scatterer.

In another preferred embodiment of the inventive photoreactor at least one of said faces (1), (2), (3), (4), (5), (6), (7) and (8) is provided with a reflector.

In the embodiment of the inventive photoreactor mentioned directly above it is preferred that each of the faces (5), (6), and (8) is provided with a reflector.

In a further preferred embodiment of the inventive photoreactor at least one of said faces (1), (2), (3), and (4) is provided with a textured surface.

In the embodiment of the inventive photoreactor mentioned directly above it is preferred that the left lateral face (1) and the right lateral face (2) are provided with a textured surface.

In the said embodiments of the inventive photoreactor exhibiting a textured surface, it is preferred that the textured surface predominantly reflects light.

In a further preferred embodiment of the inventive photoreactor said photoreactor comprises 2 to 100 reaction vessels, especially preferred 3 to 10 reaction vessels. Said multitude of reaction vessels are preferably contained in a support structure, which was described before.

Each of the at least one reaction vessel of the inventive photoreactor exhibits a front side and a rear side that are open to connect said at least one reaction vessel to devices, which are necessary to provide the at least one reaction vessel with those materials that are needed for a desired photoreaction and to harvest the desired products of said photoreaction. So, the front side and the rear side of each of the at least one reaction vessel of the inventive photoreactor are connected to pumps and other machinery, which is necessary to operate the inventive photoreactor.

In a preferred embodiment the inventive photoreactor is a bioreactor, especially a bioreactor for growing algae, for example, Spirulina algae. Such algae contain ω-3-fatty acids which - after said algae have been grown to the desired extent in the inventive photo bioreactor - are separated from the Spirulina algae.

In another preferred embodiment the inventive photoreactor is a reactor for photopolymerization, i.e. for the light-induced polymerization of monomers.

As mentioned before, the inventive photoreactor is operated, for example, with solar light or with artificially generated light. Such light sources include a more or less broad spectrum of wavelengths, whereas a certain photoreaction to be driven in the inventive photoreactor may need a special and narrow wavelength range.

Therefore, in a preferred embodiment of the inventive photoreactor, wherein the at least one reaction vessel is completely contained within the already described support structure, said support structure comprises fluorescent dye molecules which are
i) distributed within at least a part of the volume of the support structure or
ii) coated on at least a part of at least one of left lateral face (1), right lateral face (2), top lateral face (3), bottom lateral face (4), left lateral face (5), right lateral face (6), top lateral face (7) and bottom lateral face (8).

Said fluorescent dye molecules serve, for example, to shift green light in the wavelength region of 500 to 600 nm to red light in the wave length region of 600 to 700 nm, which is a favourable wavelength range to drive, for example, photosynthesis reactions.

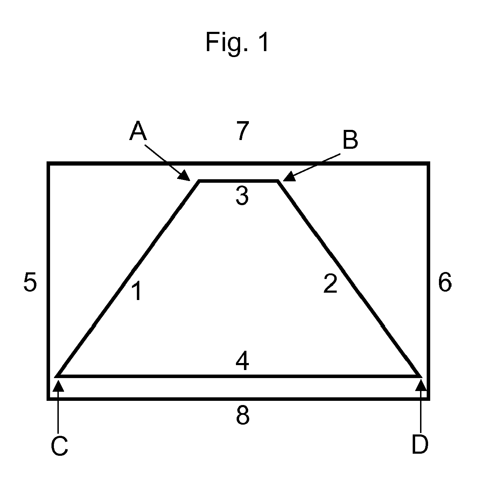

Figure 1 shows a schematic view on a preferred embodiment of the inventive photoreactor, wherein the photoreactor is viewed perpendicular to its front side. In said preferred embodiment, the inventive photoreactor comprises one reaction vessel which exhibits a trapezoidal cross-section, wherein the trapezoidal cross-section exhibits edges A, B, C and D, wherein the edges A and B are connected by a side AB, the edges C and D are connected by a side CD, the edges A and C are connected by a side AC, the edges B and D are connected by a side BD, the side AB is parallel to the side CD and the side AC is not parallel to the side BD. Said one reaction vessel exhibits a left lateral face (1), a right lateral face (2), a top lateral face (3), and bottom lateral face (4). Said one reaction vessel is completely contained in a support structure which has a cross-section in the shape of a rectangle, wherein the support structure exhibits a left lateral face (5), a right lateral face (6), a top lateral face (7), and a bottom lateral face (8).

## Claims

1. Photoreactor comprising at least one reaction vessel, wherein the at least one reaction vessel exhibits a trapezoidal cross-section, a left lateral face (1), a right lateral face (2), a top lateral face (3), and a bottom lateral face (4), and wherein at least one of the said faces (1), (2), (3) and (4) is light-transmissive.

2. Photoreactor according to claim 1, wherein the top lateral face (3) is light-transmissive.

3. Photoreactor according to claim 1, wherein each of the said faces (1), (2), (3), and (4) is light-transmissive.

4. Photoreactor according to one or more of claims 1 to 3, wherein the at least one reaction vessel is completely contained within a support structure which has a cross-section in the shape of a parallelogram, wherein the support structure exhibits a left lateral face (5), a right lateral face (6), a top lateral face (7), and a bottom lateral face (8).

5. Photoreactor according to one or more of claims 1 to 4, wherein the trapezoidal cross-section exhibits edges A, B, C and D, wherein the edges A and B are connected by a side AB, the edges C and D are connected by a side CD, the edges A and C are connected by a side AC, the edges B and D are connected by a side BD, the side AB is parallel to the side CD and the side AC is not parallel to the side BD.

6. Photoreactor according to claim 5, wherein the side AC has a length L_{AC}, the side BD has a length L_{BD}, wherein L_{AC} = L_{BD}.

7. Photoreactor according to claim 6, wherein the side AC and the side BD are shaped in the form of a number of steps, wherein the number of steps of side AC is n_{AC}, and the number of steps on side BD is n_{BD}.

8. Photoreactor according to claim 7, wherein n_{AC} = n_{BD} = n.

9. Photoreactor according to claim 8, wherein n is from 2 to 50 · 10⁶.

10. Photoreactor according to one or more of claims 1 to 9, wherein at least one of said faces (1), (2), (3), (4), (5), (6), (7) and (8) is provided with a scatterer.

11. Photoreactor according to one or more of claims 1 to 10, wherein at least one of said faces (1), (2), (3), (4), (5), (6), (7) and (8) is provided with a reflector.

12. Photoreactor according to one or more of claims 1 to 11, wherein at least one of said faces (1), (2), (3), and (4) is provided with a textured surface.

13. Photoreactor according to one or more of claim 1 to 12, wherein the photoreactor comprises 2 to 100 reaction vessels.

14. Photoreactor according to one or more of claims 1 to 13, wherein the photoreactor is a bioreactor.

15. Photoreactor according to one or more of claims 1 to 13, wherein the photoreactor is a reactor for photopolymerization.

16. Photoreactor according to one or more of claims 1 to 15, wherein the support structure comprises fluorescent dye molecules which are
i) distributed within at least a part of the volume of the support structure or
ii) coated on at least a part of at least one of left lateral face (1), right lateral face (2), top lateral face (3), bottom lateral face (4), left lateral face (5), right lateral face (6), top lateral face (7) and bottom lateral face (8).
